# EUROPEAN PATENT SPECIFICATION

(11) **EP 1 758 561 B1**
(45) Date of publication and mention of the grant of the patent: **13.06.2012**
(21) Application number: 05741811.3
(22) Date of filing: 04.05.2005
(51) Int. Cl.: A61K 9/51

(54) **PROTECTIVE MICROCONTAINERS**
SCHÜTZENDE MIKROBEHÄLTER
MICROCONTENANTS PROTECTEURS

(30) Priority: 07.05.2004 EP 04010921
(43) Date of publication of application: 07.03.2007
(73) Proprietor: Max-Planck-Gesellschaft zur Förderung der Wissenschaften e.V., 80539 München (DE)
(72) Inventor: SHCHUKIN, Dmitry, 13346 Berlin (DE); SUKHORUKOV, Gleb, Chigwell Essex, IG7 4HE (GB); MÖHWALD, Helmuth, 55411 Bingen (DE)
(74) Representative: Dey, Michael
(86) International application number: PCT/EP2005/004876
(87) International publication number: WO 2005/107679

(56) References cited:
- WO-A-00/77281
- WO-A-99/47252
- US-B1- 6 479 146
- SHCHUKIN, D.G. ET AL.: "Synthesis of binary polyelectrolyte/inorganic composite capsules of micron size" COLLOID POLYM. SCI., vol. 281, no. 12, 2003, pages 1201-1204, XP002355024
- RADTCHENKO, I.L. ET AL.: "Inorganic particle synthesis in confined micron-sized polyelectrolyte capsules" LANGMUIR, vol. 18, 2002, pages 8204-8208, XP002355028
- ANTIPOV, A.A. ET AL.: "Fabrication of a novel type of metallized colloids and hollow capsules" LANGMUIR, vol. 18, 2002, pages 6687-6693, XP002355026
- DÄHNE, L. ET AL.: "Fabrication of micro reaction cages with tailored properties" J. AM. CHEM. SOC., vol. 123, 2001, pages 5431-5436, XP002355023

## Description

The present invention relates to protective microcontainers as well as a process for preparing same. By means of the microcontainers of the invention materials enclosed therein can be protected against external influences, e.g. oxidizing or reducing agents.

Many natural and artificial objects are subjected to negative effects by the surrounding environment. It is therefore important to protect compounds which can be damaged, e.g. by irradiation, heating, oxidizing (reducing) agents, polymers, moisture, etc., on molecular level during their storage and targeted delivery. Such substances and their protection are of practical importance in medicine (drugs), gene engineering (DNA, RNA), biotechnology (proteins, individual cells, enzymes) and food industry (nutrients). Development of protective microenvironments combining defense properties with other properties, for instance, the possibility of controlled release of the secured compound at the desired site in aggressive media, is of particular interest.

Two main methodological approaches for protection of a substance include isolation of the substance inside a defensive microvolume or modification of the substance (saving desired properties if possible) with formulations or functional groups more sensitive to the certain type of aggressive treatment (such as oxidation, hydrolysis, etc.) than the protected substance itself. The latter approach was well developed and there are a number of publications describing the coupling of the compound to be protected (DNA, metal nanoparticles, quantum dots, etc.) with proteins,^{[1]} poly(amines),^{[2]} metal ions, ^{[3]} amino acids,^{[4]} surfactants,^{[5]} antioxidants,^{[6]} aromatic sulfonates, ^{[7]} chelate agents.^{[8]} Embedding of substances inside protective microvolume was shown using lipid-based microenvironments,^{[9]} liposomes,^{[10]} microbeads,^{[11]} (bio-)polymer capsules,^{[12]} amalgam capsules,^{[13]} emulsions and vesicles,^{[14]} gels^{[15]} as protective microcontainers. However, a significant shortcoming of the protective microcontainers developed so far is their monofunctionality, i.e. the protection ability cannot be combined with other properties like luminescent or magnetic functions.

Therefore, it was an object of the invention to provide microcontainers, by means of which material encapsulated therein can be protected effectively against harmful external influences. Further, the microcontainers were to have further functionalities, if necessary, besides the desired protective function.

According to the invention this object is achieved by a protective microcontainer consisting of a shell obtainable by layer-by-layer assembly of alternately charged polyelectrolytes and/or nanoparticles for protecting a material being enclosed within the microcontainer, wherein the shell comprises at least one agent which is capable of reacting with and/or converting means having an effect onto the material enclosed in the microcontainer, against which effect the material is to be protected.

According to the invention it has been found that capsules obtained by layer-by-layer assembly are excellent protective microcontainers for protecting materials encapsulated therein. In particular, desired combinations of properties can be achieved, whereby the shell can be engineered in a facile way. Layer-by-Layer (LbL) technology^{[16]} offers the opportunity to fabricate multifunctional nanoengineered microcontainers. This technology is based on sequential deposition of oppositely charged polyelectrolytes or/and nanoparticles, which allows the formation of multilayer shells from a wide range of components with nanometer precision.

The microcontainers according to the invention are protective microcontainers, i.e. that materials enclosed therein are protected against external influences by the microcontainer. The container consists of a shell which can be obtained by the Layer-by-Layer technology. The material to be protected can be provided alone or together with a suitable matrix and then coated or encapsulated respectively by layer-by-layer application of alternately charged polyelectrolytes. It is however also possible to produce hollow microcontainers by applying alternately charged polyelectrolytes and/or nanoparticles onto a core template and dissolving the core template after a shell of the desired density was formed. Such a hollow shell can afterwards be charged with the protective material, especially by increasing the permeability of the shell for the material to be inserted by external influences. Such an increase of the permeability can for example be achieved by adjusting a suitable pH-value or a corresponding salt concentration.

Furthermore, the shell of the protective microcontainers comprises at least one agent which is capable of reacting with and/or converting means having an effect onto the material enclosed in the microcontainer. The protective function of the microcontainer is provided by this agent. Said agent can be located in the shell or on the shell. Preferably, the agent is a polyelectrolyte and/or a nanoparticle, it is, however, also possible to encapsulate other substances into the shell or fix them to the shell. In a preferred embodiment, the agent reacts with the agent which would otherwise have a harmful influence on the material enclosed in the microcontainer. By reacting with the agent, the agent is rendered harmless, however, at the same time the protective agent is also stepwise used up. In a further preferred embodiment, the harmful agent is converted by the agent and thus rendered harmless without the agent itself being used up. Examples for such agents are catalysts or enzymes.

The harmful external influences, against which materials in the protective microcontainers according to the invention can be protected are for example irradiation, heat, moisture, oxidizing agents or reducing agents. Especially preferred, a protective microcontainer according to the invention is provided for the protection of a material enclosed therein from oxidizing agents, especially from low molecular weight oxidizing agents having a molecular weight of preferably < 1000 Da, more preferably < 500 Da and most preferably < 200 Da. A protection over the oxidizing effect of peroxo-compounds, especially H₂O₂ is most preferably provided.

In this case, the protective agent is preferably selected from catalysts for the decomposition of oxidizing agents such as Fe₃O₄ nanoparticles or catalase or other catalytically active nanomaterial or from sacrificial material which reacts with oxidizing agents in order to protect oxidizing agents such as peroxo-compounds.

The microcontainers according to the invention are preferably capsules. The microcontainer and preferably capsules can especially be used for encapsulating sensitive materials, e.g. drugs, nucleic acids, proteins, in particular enzymes, cells and/or food ingredients. It is possible according to the invention to encapsulate 1-100 mg material per microcontainer, more preferably 5-50 mg material per microcontainer.

An essential feature of the microcontainer according to the invnetion consists in that they can be formed multifunctionally. This means that besides the above-described protective property over an agent, further functionalities of the shell can be intended. Examples for such further functionalities are protective properties over a further harmful external influence. It is, however, also possible to give other desirable properties to the capsule as for example luminescent properties in order to enable a simple identification of the capsules, magnetic properties, sensoric properties or sustained release properties. By sustained release properties, it is possible to release the enclosed material at a desired site. Further functionalities in the shell which lead to multifunctional microcontainers can be obtained by enclosing or fixing suitable materials in or on the shell. By the layer-by-layer construction of the hollow capsule it is possible according to the invention to give desirable functionalities to the shell.

Templating of LbL polyelectrolyte films on the surface of micron and submicron-sized particles followed by their dissolution leads to formation of hollow polyelectrolyte capsules.^{[17]} The initial colloidal core determines the size of the capsules, which can be varied from 50000 nm to 10 nm, in particular, from 1000 nm to 50 nm. Most preferably capsules having a size of less than 500 nm or even less than 100 nm can be provided.

Capsules are formed by depositing alternatively charged layers of polyelectrolytes or/and nanoparticles in a layer-by-layer wise manner. Polyelectrolyte shells of this type are permeable to small molecules and ions diffusing throughout the shell by non-linear inter-site hoping^{[18]} while large molecules and nanoparticles can be entrapped inside or banned from the capsule interior. Such capsules can also be used for sustained release of encapsulated materials^{[19]}. Different additional functionalities (magnetic, luminescent, sensor) can be imparted to the shell of the capsules^{[20]}.

Due to semipermeability of polyelectrolyte shells, polyelectrolyte capsules can act as microcontainers effectively protecting encapsulated material against large polymer molecules by excluding them in outer solution^{[19a]} and, at the same time, maintaining controlled release of encapsulated low molecular weight material. *In-situ* modification of the capsule shell with inorganic nanoparticles imparts mechanical stability (nano-YF₃, nano-hydroxyapatite) and UV-protection ability (nano-TiO₂) to the polyelectrolyte shell.

According to the invention properties of the shell of LbL assembled polyelectrolyte capsules are adjusted towards protection of encapsulated material, in particular, against low molecular weight oxidative agents (such as H₂O₂), which are permeable through the capsule wall. Bovine serum albumin (BSA) was employed herein as a model protein to be protected against oxidation in the capsule interior due to the extensive characterization of its properties in reaction with peroxo radicals generated from both H₂O₂ and other peroxides.^{[21]}

Low molecular weight as used herein refers to agents, materials or substances having a molecular weight of less than 5000 Da, in particular, less than 1000 Da, more preferably less than 500 Da and most preferably less than 200 Da.

In a particularly preferred embodiment, the present invention relates to protective polyelectrolyte microcapsules that effectively prevent the oxidation of encapsulated material such as bovine serum albumin by a low molecular weight oxidative agent (such as H₂O₂). Two approaches for designing protective capsule microcontainers are demonstrated herein: The "passive armor" approach is composed of a sacrificial reducing agent as a shell constituent while the "active armor" approach includes a catalyst for decomposition of oxidative agents, in particular a catalyst for H₂O₂ decomposition deposited onto the shell as the outmost layer or included within the shell. In the latter case, protective material is not consumed during the H₂O₂ treatment, thus prolonging the duration and concentration limit of the protection activity.

The present invention further concerns a method for producing the described microcontainers and especially a process for preparing a protective microcontainer according to any of the preceding claims, wherein a hollow sphere is prepared by layer-by-layer assembly of alternately charged polyelectrolytes and/or nanoparticles, characterized in that in at least one layer the polyelectrolytes and/or nanoparticles used are an agent which reacts with or converts means having an effect on the material enclosed or to be enclosed in the microcontainer or wherein in at least one layer of the shell an agent which reacts with or converts means having an effect on the material enclosed or to be enclosed in the microcontainer is embedded.

Protective microcontainers or capsules are produced by the Layer-by-Layer technology, where alternately positively and negatively charged polyelectrolytes and nanoparticles are applied in layers. Dependent on the external influence to be defended, polyelectrolyte molecules and/or nanparticles can be chosen and inserted into the shell which react with the harmful influence or convert same. During reaction, the shell is dissolved whereas during conversion, the external influence is removed by means of enzymes or catalysts without affecting the shell itself. However, it is also possible to embed agents into the shell which react with the harmful external influence or convert same. However, the expression "embedded" comprises both the encapsulating in the shell and the fixing to the outside of the shell. In this case, protective agents can be embedded into the shell, which are different from polyelectrolytes and/or nanoparticles.

It is possible according to the invention to cover a protective material together with a matrix by layer-by-layer application of polyelectrolytes and/or nanoparticles as well as the protective agent as described herein. Alternatively, it is possible to first produce a hollow capsule and charge same with the protective material.

It is especially preferred to insert a converting agent, especially a catalyst, into the shell for the harmful external influence whereas it is possible according to the invention to provide the protective effect by inserting sacrificial material. Further, the protective agent is preferably present in or on the outer layer of the shell in order to enter a reaction with the harmful influence which is contained in the outer surrounding medium. Alternatively, it is also possible to provide the protective agent in an inner layer of the shell.

The invention further concerns the use of the above-described protective microcontainers and/or capsules for protecting a material enclosed therein of external influences and especially the use of a protecting microcontainer consisting of a shell obtainable by layer-by-layer assemly of alternately charged polyelectrolytes or/and nanoparticles to protect materials encapsulated within the shell from means having an effect onto the material, wherein in at least one layer the polyelectrolytes and/or nanoparticles used are an agent which reacts with or converts means having an effect on the material enclosed or to be enclosed in the microcontainer or wherein in at least one layer of the shell contains an agent which reacts with or converts means having an effect on the material enclosed or to be enclosed in the microcontainer is embedded.

The protective microcontainers can especially be used for protecting the materials of oxidation, in particular to protect materials from oxidation agents such as peroxo-compounds, i.e. H₂O. Materials, which can be protected, comprise pharmaceuticals, food, enzymes, compounds for the biotechnology or other sensitive substances.

Designed microcontainers can combine protective function with sustained release of the encapsulated substance and, moreover, can possess magnetic activity to perform magnetically controlled delivery of the encapsulated material in aggressive media. Protective polyelectrolyte capsules can find applications as delivery and depot systems in medicine, drug industry, and biotechnology. The protective microcontainers can further be used for preserving bioactivity of different encapsulated enzymes (urease, chymotripsin) in oxidative media.

The invention is further explained by the enclosed Figures and the following Examples.

### Figure Captions

**Figure 1****.** Confocal fluorescence microscopy image and fluorescence profile of BSA-loaded (PAH/PSS)₄-PAH polyelectrolyte capsules. Encapsulated BSA molecules were labeled with fluorescein isothiocyanate
**Figure 2****.** TEM image of Fe₃O₄-containing polyelectrolyte capsules (a) and confocal fluorescence image of (PAH/PSS)₄-PAH capsules with deposited outmost catalase layer. Catalase molecules were labeled with fluorescein isothiocyanate.
Figure 3. Oxidation of BSA encapsulated in (PAH/PSS)₄-PAH (a), (PAH/PSS)₄-PAH/catalase (b), (PAH/PSS)₄-PAH/Fe₃O₄ (c) microcapsules. Carbonyl moieties of BSA were visualized by the reaction with fluorescein isothiocyanate labeled carbazide. (d) - fluorescence profiles for a single capsule. (e) - UV/vis. spectra of encapsulated BSA derivatized with 2,4-dinitrophenylhydrazine after H₂O₂ treatment.
Figure 4. Confocal fluorescent microscopy images of (PAH/PSS)₄-PAH (a) and (PAH/PSS)₄-PAH/Fe₃O₄ (b) microcapsules in the solution containing fluorescein-labeled dextran (Mw=2000 kDa) at pH=9. Microcapsules were treated in 0.5 M H₂O₂ solution before measurements. The changes in the shell permeability are clearly seen. Scanning electron microscopy images of (PAH/PSS)₄-PAH (c) and (PAH/PSS)₄-PAH/CAT (d) microcapsules after treatment in 0.5 M H₂O₂. The differences of the shell morphology are revealed in the insets for SEM images.
**Figure 5****.** Schematic overview of the "passive" and "active" protection approaches for nanoengineered polyelectrolyte capsules.

### Examples

*Materials.* Sodium poly(styrene sulfonate) (PSS, MW - 70000), poly (allylamine hydrochloride) (PAH, MW - 50000), H₂O₂, bovine serum albumin (BSA), fluorescein-labeled bovine serum albumin (FITC-BSA), HCl, NaOH, fluorescein-labeled carbazide (FITC-carbazide), NH₃, 2,4-dinitrophenylhydrazine (DNPH), catalase (CAT), fluorescein-labeled catalase (FITC-CAT), trichloroacetic acid (TCA), Fe₂(SO₄)₃ ·7H₂O, and FeSO₄ ·7H₂O were purchased from Sigma-Aldrich. Magnetic fluid Fe₃O₄ was purchased from "Berlin Heart AG", Germany. Monodispersed MnCO₃ template particles with a diameter of 3.8 µm were synthesized as described in Ref. 22.

All chemicals were used as received. The water was prepared in a three-stage Millipore Milli-Q Plus 185 purification system and had a resistivity higher than 18 MΩ · cm.

### Example 1

### Fabrication of polyelectrolyte capsules.

Hollow PAH/PSS capsules containing 0.1 monoM of PSS monomers inside were prepared by alternated adsorption of oppositely charged PAH, PSS molecules on the surface of template MnCO₃ particles starting from a PAH layer. The resulting polyelectrolyte shell consists of 4 PAH/PSS bilayers and an outmost layer of PAH {(PAH/PSS)₄-PAH)}. After formation of PAH/PSS shells the MnCO₃ core was dissolved in 0.1 M HCl. A more detailed description of the synthesis of PAH/PSS capsules can be found elsewhere. [17]

### Example 2

### Encapsulation of bovine serum albumin.

BSA was encapsulated inside employing different permeability of the capsule shell at acidic and neutral pH.^{[19c]} Polyelectrolyte capsules were added to the acidic solution (pH=2, adjusted by HCl) containing 10 mg/ml BSA and kept for 24 hours. Then, the pH was increased to 7 by NaOH and BSA-loaded capsules were washed by deionized water. The concentration of BSA encapsulated inside the microcapsules was estimated by Lowry analysis of proteins.^{[23]}

### Example 3

### Characterization.

*Confocal microscopy.* Confocal fluorescent microscopy images of polyelectrolyte capsules in solution were obtained on a Leica TCS SP scanning system equipped with a 100x oil immersion objective and operating in fluorescein mode.

*Transmission electron microscopy.* To view the interior structure, dried polyelectrolyte capsules were embedded in gelatine holders filled with polymerized MMA (methylmethacrylate) and then cut into ultra thin sections (from 30 to 100 nm in thickness) using a Leica ultracut UCT ultramicrotome. Copper grids coated with carbon film were used to support the thin sections and a Zeiss EM 912 Omega transmission electron microscope operating at 300 kV was employed for analysis.

*Analysis of carbonyl groups.* The quantity of carbonyl groups is a valid marker to detect oxidative protein modification.^{[21]} Thus, the oxidation of BSA was monitored by the presence of carbonyl groups resulting from the reaction between protein and H₂O₂.^{[24]} Two independent analytical methods were used to measure carbonyl groups in oxidized BSA: 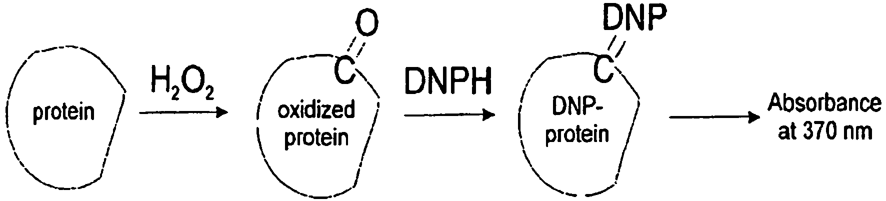
(i) oxidatively modified proteins were determined by derivatization of protein-bound carbonyl moieties with 2,4-dinitrophenylhydrazine according to Reznick and Packer.^{[24a]} For this assay, BSA-containing capsules after H₂O₂ treatment were extensively washed and protein was derivatized with DNPH overnight at room temperature. Non-encapsulated BSA oxidized in a blank experiment under the same conditions as the encapsulated one was precipitated with trichloroacetic acid, extensively washed with ethanol/ethylacetate (1:1 vol/vol), redissolved in water, and derivatized with DNPH. The resulting dinitrophenylhydrazones were determined spectrophotometrically at 370 nm using a Cary 4e spectrophotometer.
(ii) to visualize oxidized bovine serum albumin in the capsule using a confocal fluorescent microscope, FITC-labeled carbazide was utilized to label the carbonyl groups. BSA-loaded capsules after oxidation were kept in 0.05 mg/ml solution of FITC-carbazide for 4 hours at room temperature and continuous stirring. After that, the capsules were extensively washed with deionized water and studied on a confocal fluorescent microscope operating in FITC mode.

### Example 4

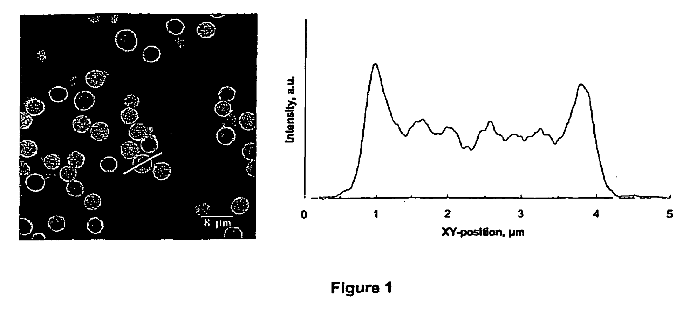

The main products of the interaction between BSA and H₂O₂ are carbonyl moieties, which can be analyzed by reliable assays. A confocal fluorescent image of polyelectrolyte capsules containing labeled FITC-BSA, which was used to prove the encapsulation procedure, is presented in Figure 1. As a result, approximately 90 % of the capsules are filled with BSA homogeneously distributed in the capsule volume (see fluorescence profile for FITC-BSA in Figure 1). Small excess at the capsule wall is caused by electrostatic interactions between BSA and charged groups of polyelectrolytes. It is important to emphasize that BSA used in further experiments was not labeled. The quantity of BSA, measured using the Lowry method,^{[23]} is 7.5 pg per capsule.

Three types of modified polyelectrolyte capsules were tested for protection properties against H₂O₂ oxidation of encapsulated BSA. As a first example, (PAH/PSS)₄-PAH capsules with PAH as the outmost layer were investigated. The presence of poly(allylamine) in the shell anticipates the antioxidant properties of the shell because PAH can be irreversibly oxidized by H₂O₂, thus forming a sacrificial barrier for H₂O₂ going through the shell inside the capsule. Two other kinds of protective microcapsules are (PAH/PSS)₄-PAH capsules surface-engineered by depositing a catalyst of H₂O₂ decomposition to H₂O and less active O₂ as the outer layer. Nanosized Fe₃O₄ and the enzyme catalase were chosen as catalysts for shell modification.

Fe₃O₄ nanoparticles were introduced in the polyelectrolyte shell before BSA encapsulation by either adsorption of pre-formed Fe₃O₄ from magnetic fluid or *in-situ* synthesis of nano-Fe₃O₄ directly in the shell from a solution of 0.1 M FeSO₄ + 0.1 M Fe₂(SO₄)₃ according to the procedure described in Ref. 25. The presence of nanosized Fe₃O₄ is seen on the TEM image of ultramicrotomed polyelectrolyte capsules. Dense arrangement of Fe₃O₄ nanoparticles in the polyelectrolyte shell was observed and no magnetite precipitate was detected in the capsule volume. Formation of magnetite nanoparticles during *in-situ* synthesis in the capsule shell was confirmed by wide angle X-ray scattering. The size of the Fe₃O₄ nanoparticles is around 4 nm.

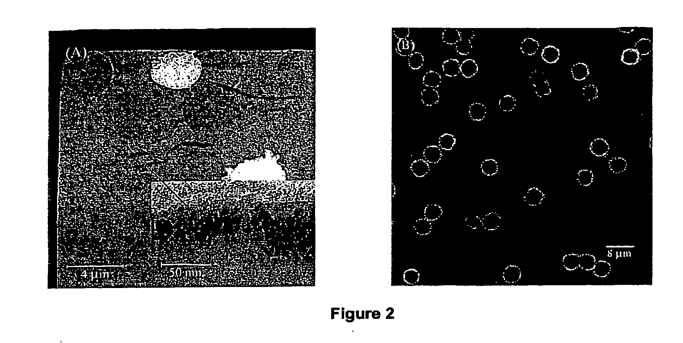

In another case, the deposition of catalase on the surface of positively charged (PAH/PSS)₄-PAH capsules was performed at a pH value above the isoelectric point of the enzyme to give catalase molecules a negative charge. The outmost catalase layer was formed from 5 mg/ml CAT solution at pH=7 and 20 min of incubation. Then, the rest of undeposited catalase was washed out. Visualization of the catalase layer on the surface of the polyelectrolyte capsules was made using labeled FITC-CAT. As seen in Figure 2b, a homogeneous catalase layer was obtained.

Polyelectrolyte capsules loaded with unlabeled BSA were added to 0.005 M solution of H₂O₂ for 30 min. For comparison, an equivalent quantity of free BSA was also exposed to H₂O₂. The quantity of the resulting carbonyl groups was measured by their derivatization with 2,4-dinitrophenylhydrazine and visualized in confocal fluorescent microscope by reaction with FITC-carbazide.

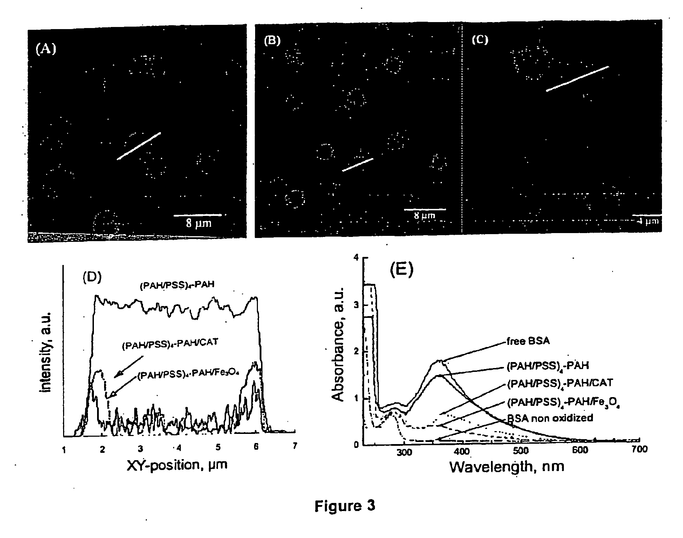

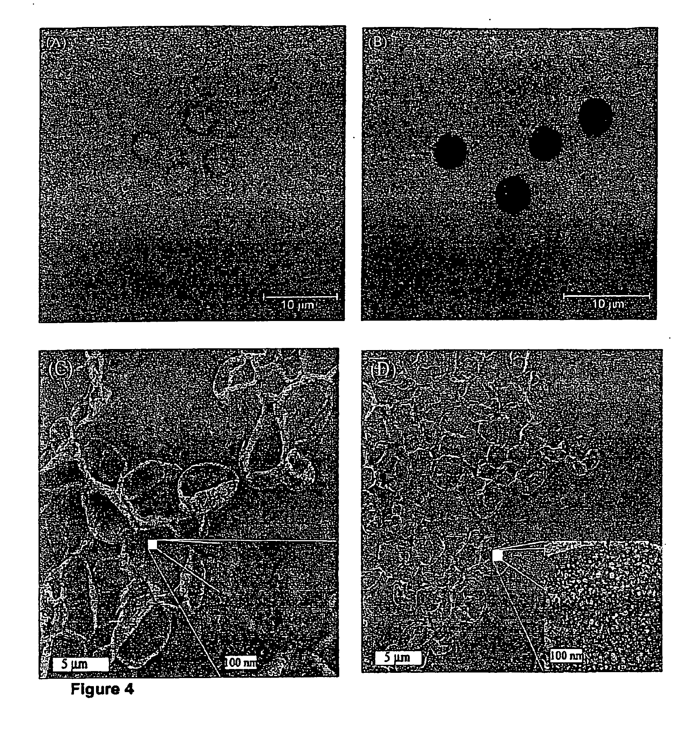

Initial (PAH/PSS)₄-PAH shell reveals small protection effect caused by the partial reduction of H₂O₂ molecules, which penetrates the polyelectrolyte shell, by amino groups of PAH (Fig. 3). Sacrificial oxidation of PAH leads to loss of integrity, controlled permeability properties, and, as a consequence, to destruction of the polyelectrolyte shell at high H₂O₂ concentrations or prolonged H₂O₂ treatment (Fig. 4). (PAH/PSS)₄-PAH polyelectrolyte capsules become permeable to fluorescein-labeled dextran of Mw=2000000 at alkali pH=9 after treatment in 0.05 M H₂O₂ (Fig. 4a) while initial (PAH/PSS)₄-PAH capsules (before oxidation) are closed for dextran molecules of 2000 kDa in alkali pH.^{[18]} Formation of cracks and holes in the polyelectrolyte shell was found in 0.05 M H₂O₂ solution (Fig. 4c). The protection efficiency can be increased by doping the polyelectrolyte shell with an electron donor better than PAH (e.g. polyvinylalcohol^{[26]}), however, the limited protection capacity, described above, still remains.

One of the possible approaches to eliminate this drawback is to create an "active" barrier on the surface of the (PAH/PSS)₄-PAH microcapsule, i.e. to combine a passive redox mechanism with an active catalytic protection. Modification of polyelectrolyte shell with Fe₃O₄ or CAT, which catalyzes H₂O₂ decomposition, results in a drastic increase in antioxidant properties of the shell and decreases a number of carbonyl moieties of encapsulated BSA after H₂O₂ treatment (Fig. 3b-e). The most effective is a nano-Fe₃O₄ layer, similar protection yield was observed for both pre-formed and *in-situ* synthesized Fe₃O₄. The catalase-modified polyelectrolyte capsule is 1.7 times less effective as compared to Fe₃O₄-modified, possibly due to partial deactivation of the enzyme after adsorption on PAH and oxidation by OH mediates of H₂O₂ decomposition on catalase. Addition of the active outmost layer increases the stability and integrity of the capsule shell (Fig. 4d), they save nonlinear inter-site hoping^{[18]} permeability properties for high molecular weight molecules remaining in the "dose" state for dextran of 2000 kDa at pH=9 (Fig. 4b).

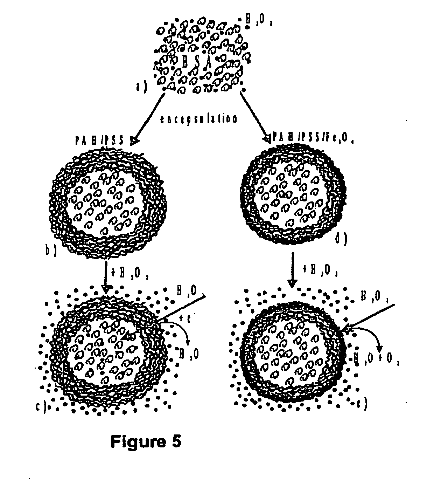

Both types of protective mechanisms are schematically represented in Figure 5. For "passive armor" shells part of H₂O₂ is reduced by PAH while moving through the shell toward encapsulated BSA. "Active armor" combines catalytic and redox mechanisms providing a more efficient protection barrier, thus increasing the H₂O₂ concentration limits and duration time, at which encapsulated BSA is protected against oxidation. The magnetic activity of polyelectrolyte capsules doped with Fe₃O₄ is also preserved after (and during) H₂O₂ treatment.

### References:

[1] a) Y. Yamamoto, L.B. Poole, R.R. Hantgan J. Bacteriol. 2002, 184, 2931. b) R.A. Grant, D.J. Filman, S.E. Finkel, Nat. Struct. Biol. 1998, 5, 294. c) A. Martinez, R. Kolter, J. Bacteriol. 1997, 179, 5188.
[2] a) S. Katayose, K. Kataoka, Bioconjugate. Chem. 1997, 8, 702. b) N.P.M. Smit, A.A. Vink, R.M. Kolb, Photochem. Photobiol. 2001, 74, 424. c) L. D'Agostino, A.A. Di Luccia, Eur. J. Biochem. 2002, 269, 4317.
[3] a) M.T. Leccia, M.J. Richard, A. Favier, Biol. Trace Elem. Res. 1999, 69, 177. b) H.B. Ambroz, T.J. Kemp, E.M. Komacka, Radiat. Phys. Chem. 1998, 53, 491.
[4] S. Kanvah, G.B. Schuster, J. Am. Chem. Soc. 2002, 124, 11286.
[5] H. Ichikawa, N. limura, H. Wakabayashi, J. Colloid Interf. Sci. 2000, 228, 32.
[6] a) M. Dansapetretski, J.M.C. Ribeiro, G.C. Atella J. Biol. Chem. 1995, 270, 10893. b) A. Salvi, C. Bruhlmann, E. Migliavacca, Helv. Chim. Acta 2002, 85, 867. c) J.C. Mayo, D.X. Tan, R.M. Sainz, BBA-Gen. Subjects 2003, 1620, 139.
[7] D. Matulis, C. Wu, T.V. Pham, J. Mol. Catal. B-Enzym. 1999, 7, 21.
[8] J.G. Correa, A.O.M. Stoppani, Free Radical Res. 1996, 24, 311.
[9] V. Jenning, S.H. Gohla, J. Microencapsul. 2001, 18, 149.
[10]D.H. Chen, D.L. Cole, G.S. Srivastsa, J. Pharmaceut. Biomed. 2000, 22, 791.
[11]a) D. Quong, R.J. Neufeld, J. Microencapsul. 1999, 16, 573. b) J.P. Liu, R.O. Williams, Eur. J. Pharm. Biopharm. 2002, 53, 167.
[12]a) M. Dunne, D.C. Bibby, J.C. Jones, J. Control Release 2003, 92, 209. b) S. Darquy, M.E. Pueyo, F. Capron, Artif. Organs 1994, 18, 898. c) S.K. Korner, F.C. Tucci, D.M. Rudkevich, Chem-Eur. J. 2000, 6, 187.
[13]M.E. Stone, E.D. Pederson, M.E. Cohen, Dent. Mater. 2002, 18, 289.
[14]M. Genty, G. Couarraze, R. Laversanne, J. Control. Release 2003, 90, 119.
[15]C. Q. Qin, L. Xiao, Y.M. Du, React. Funct. Polym. 2002, 50, 165.
[16]a) G. Decher, J.D. Hong, J. Schmitt, Thin Solid Films 1992, 210/211, 831. b) G. Decher, Science 1997, 277, 1232.
[17]a) G.B. Sukhorukov, E. Donath, S. Davis, H. Lichtenfeld, F. Caruso, V.I. Popov, H. Möhwald, Polym. for Adv. Tech. 1998, 9, 759. b) E. Donath, G.B. Sukhorukov, F. Caruso, S. Davis, H. Möhwald, Angew. Chem.-Int. Ed. 1998, 37, 2202.
[18]a) T.R. Farhat, J.B. Schlenoff, J. Am. Chem. Soc. 2003, 125, 4627. b) A.A. Antipov, G.B. Sukhorukov, H. Möhwald, Langmuir, 2003, 19, 2444.
[19]a) G.B. Sukhorukov, M. Brumen, E. Donath, H. Möhwald, J. Phys. Chem. B 1999, 103, 6434. b) H. Ai, S.A. Jones, M.M. de Villiers, Y.M. Lvov, J. Control. Release 2003, 86, 59. c) A.A. Antipov, G.B. Sukhorukov, S. Leporatti, I.L. Radtchenko, E. Donath, H. Möhwald, Colloid Surface A 2002, 198, 535.
[20]a) D.G. Shchukin, I.L. Radtchenko, G.B. Sukhorukov, Mater. Lett. 2003, 57, 1743. b) N. Gaponik, I.L. Radtchenko, G.B. Sukhorukov, H. Weller, A.L. Rogach, Adv. Mater. 2002, 14, 879.
[21]a) A.A. Platt, S.P. Gieseg, Redox Rep. 2003, 8, 81. b) E. Damiani, C. Belaid, P. Carloni, L. Greci, Free Radical Res. 2003, 37, 731.
[22]A.A. Antipov, D. Shchukin, Y. Fedutik, A.I. Petrov, G.B. Sukhorukov, H. Möhwald, Colloid Surface A 2003, 224, 175.
[23]O.H. Lowry, N.J. Rosebrough, A.L. Farr, R.J. Randall, J. Biol. Chem. 1951, 193, 265.
[24]a) A. Reznick, L. Packer, **1994,** *233*, 357. b) R.L. Levine, J.A. Williams, E.R. Stadtman, E. Shacter, Methods Enzymol. 1994, 233, 346.
[25]D.G. Shchukin, I.L. Radtchenko, G.B. Sukhorukov, J. Phys. Chem. B 2003, 107, 952.
[26]D.G Shchukin, E. Ustinovich, D.V. Sviridov, Y.M. Lvov, G.B. Sukhorukov, Photochem. Photobiol. Sci. 2003, 2, 975.

## Claims

1. Protective microcontainer consisting of a shell obtainable by layer-by-layer assembly of alternately charged polyelectrolytes and/or nanoparticles for protecting a material being enclosed within the microcontainer, wherein the shell comprises in or on the outmost layer at least one agent which is capable of reacting with and/or converting means having an effect onto the material enclosed in the microcontainer, against which effect the material is to be protected.

2. Protective microcontainer according to claim 1, wherein the means having an effect are selected from irradiation, heat, moisture, oxidizing agents and/or reducing agents, in particular, from oxidizing or reducing agents.

3. Protective microcontainer according to claim 1 or 2, wherein the means having an effect onto the enclosed material are oxidizing agents, in particular, low-molecular weight oxidizing agents such as H₂O₂.

4. Protective microcontainer according to any of the preceding claims, wherein the agent which reacts with or converts means having an effect on the enclosed material is selected from sacrificial material and/or catalysts.

5. Protective microcontainer according to any of the preceding claims wherein the material enclosed in the microcontainer is selected from drugs, nucleic acids, proteins, in particular, enzymes, cells and/or food ingredients.

6. Protective microcontainer according to any of the preceding claims, having a size from 50 nm to 50,000 nm.

7. Protective microcontainer according to any of the preceding claims, wherein the shell contains in or on the outmost layer an agent which is capable of preventing oxidation of the encapsulated material, which agent is selected from catalysts for decomposition of oxidizing agents, in particular, of low molecular weight oxidizing agents.

8. Protective microcontainer according to claim 7, wherein the agent is selected from Fe₃O₄ nanoparticles or catalase.

9. Protective microcontainer according to any of the preceding claims, being a multifunctional microcontainer.

10. Process for preparing a protective microcontainer according to any of the preceding claims, wherein a hollow sphere is prepared by layer-by-layer assembly of alternately charged polyelectrolytes and/or nanoparticles, **characterized in that** in or on the outmost layer the polyelectrolytes and/or nanoparticles used are an agent which reacts with or converts means having an effect on the material enclosed or to be enclosed in the microcontainer or wherein in or on the outmost layer of the shell an agent which reacts with or converts means having an effect on the material enclosed or to be enclosed in the microcontainer is embedded.

11. Process according to claim 10, wherein the hollow capsule is loaded with a material.

12. Process according to any of the preceding claims, wherein a catalyst for decomposition of means having an effect on the materials enclosed or to be enclosed in the microcontainer is embedded in or on the outmost layer of the shell.

13. Use of a protecting microcontainer consisting of a shell obtainable by layer-by-layer assemly of alternately charged polyelectrolytes or/and nanoparticles to protect materials encapsulated within the shell from means having an effect onto the material, wherein in or on the outmost layer the polyelectrolytes and/or nanoparticles used are an agent which reacts with or converts means having an effect on the material enclosed or to be enclosed in the microcontainer or wherein in or on the outmost layer of the shell an agent which reacts with or converts means having an effect on the material enclosed or to be enclosed in the microcontainer is embedded.

14. Use according to claim. 13 to protect materials from oxidation or reduction, in particular, to protect materials from oxidizing agents.

15. Use according to claim 13 or 14, wherein the microcontainer comprises in or on the outmost layer of the shell a sacrificial material and/or a catalyst for reacting with means having an effect on the enclosed material, in particular, for decomposition of oxidizing agents.

16. Use according to any of claims 13-15, wherein the microcontainer comprises in or on the outmost layer of the shell Fe₃O₄ nanoparticles or catalase.

17. Protective microcontainer according to any of claims 1-10 for use in therapy as delivery and/or depot system.

## Patentansprüche

1. Schützender Mikrobehälter bestehend aus eine Hülle, erhältlich durch Schicht-für-Schicht-Anordnung von alternierend geladenen Polyelektrolyten und/oder Nanopartikeln zum Schutz eines Materials, welches innerhalb des Mikrobehälters eingeschlossen ist, wobei die Hülle in oder auf der äußersten Schicht mindestens einen Wirkstoff umfasst, welcher in der Lage ist mit Mitteln zu reagieren und/oder umzuwandeln, die eine Wirkung auf das eingeschlossene Material im Mikrobehälter aufweisen, gegen welche Wirkung das Material zu beschützen ist.

2. Schützender Mikrobehälter nach Anspruch 1, wobei die Mittel, die eine Wirkung aufweisen ausgewählt sind aus Bestrahlung, Hitze, Feuchtigkeit, Oxidationsmittel und/oder Reduktionsmitteln, insbesondere aus Oxidations- oder Reduktionsmitteln.

3. Schützender Mikrobehälter nach Anspruch 1 oder 2, wobei die Mittel; die eine Wirkung auf das eingeschlossene Material aufweisen, Oxidationsmittel sind, insbesondere Oxidationsmittel mit niedrigem Molekulargewicht wie H₂O₂.

4. Schützender Mikrobehälter nach einem der vorhergehenden Ansprüche, wobei der Wirkstoff, welcher mit den Mitteln reagiert oder diese umwandelt, einer Wirkung auf das eingeschlossene Material aufweist, ausgewählt ist aus Opfermaterial und/oder Katalysatoren.

5. Schützender Mikrobehälter nach einem der vorhergehenden Ansprüche, wobei das Material, welches in den Mikrobehälter eingeschlossen ist ausgewählt ist aus Arzneimitteln, Nukleinsäuren, Proteinen, insbesondere Enzymen, Zellen und/oder Lebensmittel-Inhaltssfoffen.

6. Schützender Mikrobehälter nach einem der vorhergehenden Ansprüche, der eine Größe von 50 nm bis 50,000 nm aufweist.

7. Schützender Mikrobehälter nach einem der vorhergehenden Ansprüche, wobei die Hülle in oder auf der äußersten Schicht einen Wirkstoff enthält, welcher in der Lage ist, Oxidation des eingekapselten Materials zu verhindern, wobei der Wirkstoff ausgewählt ist aus Katalysatoren für die Zersetzung von Oxidationsmitteln, insbesondere von Oxidationsmitteln mit niedrigem Molekulargewicht.

8. Schützender Mikrobehälter nach Anspruch 7, wobei der Wirkstoff ausgewählt ist aus Fe₃O₄ Nanopartikeln oder Katalase.

9. Schützender Mikrobehälter nach einem der vorhergehenden Ansprüche, wobei er ein multifunktionaler Mikrobehälter ist.

10. Verfahren zur Herstellung eines schützenden Mikrobehälters nach einem der vorhergehenden Ansprüche, wobei eine hohle Kugel durch Schicht-für-Schicht-Anordnung von alternierend geladenen Polyelektrolyten und/oder Nanopartikeln hergestellt wird, **dadurch gekennzeichnet, dass** in oder auf der äußersten Schicht die verwendeten Polyelektrolyte und/oder Nanopartikeln ein Wirkstoff sind, welche mit Mitteln reagieren oder diese umwandeln, welche eine Wirkung auf das eingeschlossene oder das in den Mikrobehälter einzuschließende Material aufweisen oder wobei in oder auf der äußersten Schicht der Hülle ein Wirkstoff, welcher mit den Mitteln reagiert oder diese umwandelt, welche eine Wirkung auf das eingeschlossene oder das in den Mikrobehälter einzuschließende Material aufweisen, eingebettet ist.

11. Verfahren nach Anspruch 10, wobei die hohle Kapsel mit einem Material beladen ist.

12. Verfahren nach einem der vorhergehenden Ansprüche, wobei ein Katalysator für die Zersetzung von Mitteln, die eine Wirkung auf die eingeschlossen oder in der Mikrobehälter einzuschließenden Material aufweisen, in oder auf die äußerste Schicht der Hülle eingebettet ist.

13. Verwendung eines schützenden Mikrobehälters bestehend aus einem Hülle, erhältlich durch Schicht-für-Schicht-Anordnung von alternierend geladenen Polyelektrolyten oder/und Nanopartikeln, um innerhalb der Schale eingekapselte Materialien vor Mitteln zu schützen, welche eine Wirkung auf das Material aufweisen, wobei in oder auf der äußersten Schicht die verwendeten Polyelektrolyte und/oder Nanopartikel ein Wirkstoff sind, welche mit Mitteln reagieren oder umwandeln, welche eine Wirkung auf das eingeschlossen oder das in den Mikrobehälter einzuschließende Material aufweisen oder wobei in oder auf der äußersten Schicht der Hülle ein Wirkstoff, welcher mit den Mitteln reagiert oder diese umwandelt, welche eine Wirkung auf das eingeschlossene oder in den Mikrobehälter einzuschließende Material aufweisen, eingebettet ist.

14. Verwendung nach Anspruch 13, um Materialien vor Oxidation oder Reduktion zu schützen, insbesondere, um Materialien vor Oxidationsmitteln zu schützen.

15. Verwendung nach Anspruch 13 oder 14, wobei der Mikrobehälter in oder auf der äußersten Schicht der Hülle ein Opfermaterial und/oder einen Katalysator für die Reaktion mit Mitteln umfasst, die eine Wirkung auf das eingeschlossene Material, insbesondere für die Zersetzung von Oxidationsmitteln aufweisen.

16. Verwendung nach einem der Ansprüche 13-15, wobei der Mikrobehälter in oder auf der äußersten Schicht der Hülle Fe₃0₄ Nanopartikel oder Katalase umfasst.

17. Schützender Mikrobehälter nach einem der Ansprüche 1-10 zur Verwendung in Therapie als Abgabe- und/oder Depot-System.

## Revendications

1. Micro-contenant protecteur consistant en une coque pouvant être obtenue par un assemblage couche-par-couche de polyélectrolytes et/ou de nanoparticules chargés alternativement, pour la protection d'un matériau à enfermer dans le micro-contenant, où la coque comprend dans ou sur la couche la plus extérieure, au moins un agent qui est capable de réagir avec et/ou de convertir des moyens ayant un effet sur le matériau enfermé dans le micro-contenant, effet contre lequel le matériau doit être protégé.

2. Micro-contenant protecteur selon la revendication 1, où les moyens ayant un effet sont sélectionnés parmi une irradiation, la chaleur, l'humidité, des agents oxydants et/ou des agents réducteurs, en particulier des agents oxydants ou réducteurs.

3. Micro-contenant protecteur selon la revendication 1 ou 2, où les moyens ayant un effet sur le matériau enfermé sont des agents oxydants, en particulier des agents oxydants de faible poids moléculaires tels H₂O₂.

4. Micro-contenant protecteur selon l'une quelconque des revendications précédentes, où l'agent qui réagit avec ou qui convertit les moyens ayant un effet sur le matériau enfermé, est choisi parmi un matériau sacrificiel et/ou des catalyseurs.

5. Micro-contenant protecteur selon l'une quelconque des revendications précédentes, où le matériau enfermé dans le micro-contenant est sélectionné parmi des médicaments, des acides nucléiques, des protéines, en particulier des enzymes, des cellules et/ou des ingrédients alimentaires.

6. Micro-contenant protecteur selon l'une quelconque des revendications précédentes, ayant une taille située dans l'intervalle allant de 50 nm à 50 000 nm.

7. Micro-contenant protecteur selon l'une quelconque des revendications précédentes, où la coque contient dans ou sur la couche la plus extérieure, un agent qui est capable de prévenir l'oxydation du matériau encapsulé, lequel agent est choisi parmi les catalyseurs pour la décomposition des agents oxydants, en particulier les agents oxydants de faible poids moléculaire.

8. Micro-contenant protecteur selon la revendication 7, où l'agent est choisi parmi des nanoparticules de Fe₃O₄ ou une catalase.

9. Micro-contenant protecteur selon l'une quelconque des revendications précédentes, qui est un micro-contenant multifonctionnel.

10. Procédé de préparation d'un micro-contenant protecteur selon l'une quelconque des revendications précédentes, où une sphère creuse est préparée par assemblage couche-par-couche de polyélectrolytes et/ou nanoparticules chargés alternativement, **caractérisé en ce que** dans ou sur la couche la plus extérieure, les polyélectrolytes et/ou nanoparticules utilisés sont un agent qui réagit avec ou qui convertit des moyens ayant un effet sur le matériau enfermé ou à enfermer dans le micro-contenant, ou où dans ou sur la couche la plus extérieure de la coque, un agent qui réagit avec ou qui convertit des moyens ayant un effet sur le matériau enfermé ou à enfermer dans le micro-contenant.

11. Procédé selon la revendication 10, où la capsule creuse est chargée d'un matériau.

12. Procédé selon l'une quelconque des revendications précédentes, où un catalyseur pour la décomposition des moyens ayant un effet sur les matériaux enfermés ou à enfermer dans le micro-contenant, est piégé dans ou sur la couche la plus extérieure de la coque.

13. Utilisation d'un micro-contenant consistant en une coque pouvant être obtenue par un assemblage couche-par-couche de polyélectrolytes et/ou de nanoparticules chargés alternativement, pour la protection de matériaux encapsulés dans la coque, de moyens ayant un effet sur le matériau, où dans ou sur la couche la plus extérieure, les polyélectrolytes et/ou les nanoparticules utilisés sont un agent qui réagit avec ou qui convertit les moyens ayant un effet sur le matériau enfermé ou à enfermer dans le micro-contenant, ou où dans ou sur la couche la plus extérieure de la coque, un agent qui réagit avec ou qui convertit les moyens ayant un effet sur le matériau enfermé ou à enfermer dans le micro-contenant.

14. Utilisation selon la revendication 13, pour protéger des matériaux de l'oxydation ou de la réduction, en particulier pour protéger des matériaux d'agents oxydants.

15. Utilisation selon la revendication 13 ou 14, où le micro-contenant comprend dans ou sur la couche la plus extérieure de la coque, un matériau sacrificiel et/ou un catalyseur pour réagir avec les moyens ayant un effet sur le matériau enfermé, en particulier pour décomposer les agents oxydants.

16. Utilisation selon l'une quelconque des revendications 13 à 15, où le micro-contenant comprend dans ou sur la couche la plus extérieure de la coque, des nanoparticules de Fe₃O₄ ou une catalase.

17. Micro-contenant protecteur selon l'une quelconque des revendications 1 à 10, à utiliser en thérapie, comme système de délivrance et/ou de dépôt.
